Europäisches Patentamt

European Patent Office

Office européen des brevets

(11) Publication number: **0 406 014 A1**

# EUROPEAN PATENT APPLICATION

(21) Application number: **90307161.1**

(22) Date of filing: **29.06.90**

(51) Int. Cl.⁵: **A61B 17/064**

(30) Priority: **30.06.89 US 374675**

(43) Date of publication of application:
**02.01.91 Bulletin 91/01**

(84) Designated Contracting States:
**BE DE DK ES FR GB IT LU NL**

(71) Applicant: **ETHICON INC.**
**U.S. Route 22**
**Somerville New Jersey 08876(US)**

(72) Inventor: **Mills, Earl J.**
**2801 Whitehouse Lane**
**Cincinnati, Ohio 45244(US)**
Inventor: **Bendel, Lee P.**
**28 Country Hill Road**
**Lebanon, New Jersey 08833(US)**
Inventor: **Sardelis, Timothy A.**
**Old Lane Highwood**
**Somerset, New Jersey 08873(US)**

(74) Representative: **Fisher, Adrian John et al**
**CARPMAELS & RANSFORD 43 Bloomsbury**
**Squareare**
**London WC1A 2RA(GB)**

(54) **Image enhancing surgical staple.**

(57) A biocompatible metallic surgical staple formed from a non-magnetic components and comprised of more than 50% titanium by weight is described. The staple is formed from at least 90% titanium and includes a 6% aluminum and 4% vanadium. It has been found that the low density, high strength and non-magnetic properties of the present staple make it desirable for magnetic-resonance imaging scanning.

# FIG. 1

DIRECTION OF FORMING — 10

EP 0 406 014 A1

## IMAGE ENHANCING SURGICAL STAPLE

### Field of the Invention

This invention relates generally to improved surgical staples. More specifically, this invention relates to improved surgical staples containing inert, non-magnetic, imaging enhancing materials. Most specifically, this invention relates to improved titanium surgical staples.

### Background of the Invention

Surgical staples have been in use for quite some time. Certain regular problems have been encountered by surgeons using conventional surgical staples.

First, surgical staples have usually been formed from either stainless steel or a bioabsorbable polymer. In steel, a material high in density, the staples are difficult to form or drive into skin. These staples, which have a size between .008 inches diameter and .020 inches diameter, require at forces in the range of 40 pounds to 100 pounds to form in skin tissue. Naturally, because mechanical advantage is difficult to derive in a surgical stapler, this requires increased stapling force imparted by the user.

On the other hand, bioabsorbable materials are generally so low in density that they are virtually impossible to form, because any bending force will cause them to shear. Thus, bioabsorbable polymers generally require the placement of receivers in order to retain the (preformed) staples.

Present staples further can sometimes be difficult to enhance during a CAT-scan imaging process, because the staples are, for one reason or another, difficult to detect. Specifically, present staples may cause "starburst" imaging patterns. Thus, one reading a CAT-scan has a difficult time determining exact placement of the staple and, of course, surrounding tissue images are obscured by the "starburst" pattern.

Also, because stainless steel staples can have magnetic properties, during magnetic-resonance imaging (MRI) there is greater "artifact" or warping of images, by the staples in the imaging receiver. Accordingly, MRI is difficult to perform with stainless steel staples.

Finally, of course, it is a requirement that staples be inert to body tissue. Generally with bioabsorbable material or stainless steel material, the components must be chosen carefully in order to properly configure an inert staple, which will avoid allergic responses.

### Summary of the Invention

It is therefore an object of the invention to provide a low density, high strength material for use in surgical staples.

It is a further object of the present invention to provide low density, high strength staples which allow imaging enhancement during CAT-scans.

It is yet a further object of the present invention to provide a surgical staple formed from a non-magnetic material, resulting in less "artifact", such that said staple can be used during MRI processes.

Finally, it is an object of the present invention to provide a staple having inert and biocompatible properties.

These and other objects of the present invention are accomplished in a surgical staple containing non-magnetic components and comprised of at least 50% titanium by weight. In particular, it has been found that a surgical staple formed from 90% titanium most beneficially enhances all the desired properties of the present invention.

### Detailed Description of the Drawings

The present invention is described in the accompanying drawings in which:

Fig. 1 is a side view of an unformed typical ligating clip; and

Fig. 2 is a side view of an unformed typical surgical staple.

### Detailed Description of the Invention

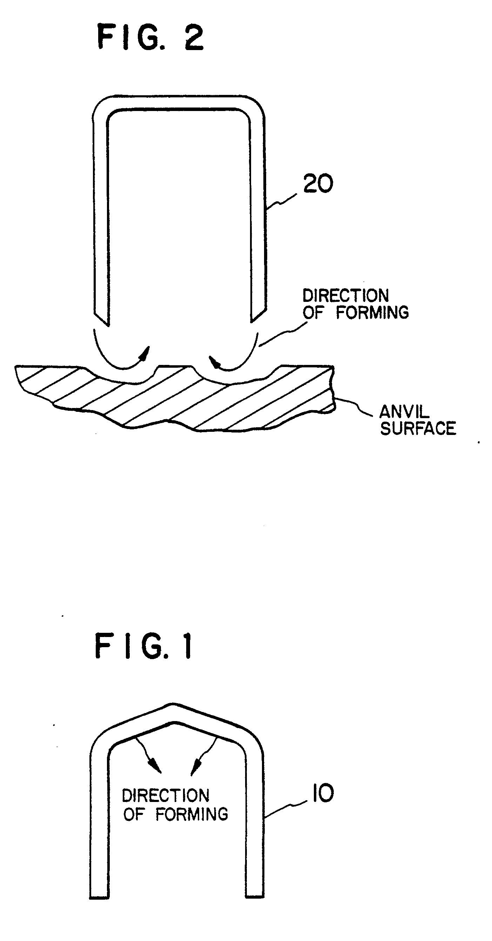

As can be seen from Fig. 1, a ligating clip 10 generally contains a pair of folded sides 12. These ligating clips generally have a diameter similar to surgical staples, that is, between .008″ and .022″. Accordingly, when a lateral force is applied to these folded sides, much of the necessary bend already exists in the ligating clip 10. Thus, the amount of force necessary to form a typical ligating clip around a blood vessel is in the range of approximately 20 pounds to 40 pounds.

Yet, surgical staples must generally be formed around and through tissue. As seen in Fig. 2, the staple 20 is generally bent about an anvil or is preformed and placed around tissue within the body. Because staples are generally placed in large arrays of rows or circles within a stapler, the forces to fire these staples are usually much higher than those used to form single or double ligating clips.

Titanium has been used as an element in surgical ligating clips. Yet, because the necessary forces imparted on ligating clips are extremely different from the forces imparted on surgical staples, titanium has been difficult to develop as a material used in surgical staples. Specifically, because titanium clips are virtually fully preformed, less springback results in the forming of a titanium clip. On the other hand, titanium staples must go through full deformation to be properly formed. During such full deformation, prototype pure titanium staples would partially become displaced from the tissue or, worse, "springback" to a partially open portion or buckle and even collapse before the staple was fully formed.

Heretofore, a compromise was reached concerning surgical staples. That is, these staples were formed from stainless steel, which has a higher bending modulus and has less "springback" after formation. The sacrifice was the creation of a staple with a generally higher inherent force required to form the surgical staple. However, because the stainless steel staple remained closed, it was more desirable as a surgical staple. Unfortunately, surgical staples formed from stainless steel maintain characteristics that are not necessarily desirable for staples, such as reduced image enhancement qualities.

The present surgical staples are formed from titanium alloys. They contain at least 50% titanium, and more preferably from 50 to 98% titanium. Even more preferably, the staples comprise from 85 to 95% titanium, e.g. from 85 to 95% titanium. Preferably, the balance of the titanium alloy is aluminum and/or vanadium, together with unavoidable impurities. particularly preferred alloys contain from 2 to 12% aluminum and from 1 to 8% vanadium by weight, e.g. from 3 to 9% aluminum and from 2 to 6% vanadium. It has been found that an alloy of about 90% titanium with about 6% aluminum and about 4% vanadium is most desirable. while pure titanium staples are difficult to produce, the alloy is much simpler to use in staple manufacture. The alloy tests effectively for production as well as in CAT-scan imaging, and magnetic-resonance imaging.

Specifically, it has been found that this alloy results in a surgical staple which does not "springback" after forming. This is due to the higher elastic modulus and cold working behavior of the titanium alloy used in the surgical staple.

Titanium is a low density material and therefore requires less force to form than present stainless steel staples. It is lightweight and yet, strong, durable and pliable, allowing desirable properties to be formed in a single staple. Especially important is the effectiveness of the titanium staple which also allows enhanced MRI properties, previously not possible with stainless steel staples.

While the invention has been described in connection with the present preferred embodiment, it will be understood that variations are possible within the scope of the attached claims.

## Claims

1. A biocompatible metallic surgical staple formed from non-magnetic components and comprised of more than 50% titanium by weight.

2. A staple according to claim 1 comprising from 50 to 98% titanium by weight.

3. A staple according to claim 1 or claim 2 wherein the balance is aluminum and/or vanadium, plus unavoidable impurities.

4. A staple according to any preceding claim comprising from 80 to 95% titanium by weight.

5. A staple according to claim 4 comprising from 2 to 12% aluminum and from 1 to 8% vanadium by weight.

6. A staple according to claim 5 comprising from 85 to 95% titanium, from 3 to 9% aluminum and from 2 to 6% vanadium by weight.

7. A staple according to claim 6 comprising about 90% titanium, about 6% aluminum and about 4% vanadium by weight.

# FIG. 2

20

DIRECTION
OF FORMING

ANVIL
SURFACE

# FIG. 1

DIRECTION
OF FORMING

10

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 399 810 (SAMUELS et al.)<br>* Column 2, lines 38-41; figure 5 *<br>--- | 1,2 | A 61 B 17/064 |
| A | US-A-4 765 335 (SCHMIDT et al.)<br>* Column 2, lines 62-66; column 5, lines 13-22 *<br>--- | 1,2 | |
| A | EP-A-0 040 884 (SMIT)<br>* Page 6, lines 18-22; figure 1 *<br>----- | 3 | |
| | | | TECHNICAL FIELDS SEARCHED (Int. Cl.5)<br><br>A 61 B |

The present search report has been drawn up for all claims

| Place of search | Date of completion of the search | Examiner |
|---|---|---|
| THE HAGUE | 02-10-1990 | MOERS R.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)